# EUROPEAN PATENT SPECIFICATION

(11) **EP 0 472 315 B1**
(45) Date of publication and mention of the grant of the patent: **19.10.1994**
(21) Application number: 91307125.4
(22) Date of filing: 02.08.1991
(51) Int. Cl.: A61F 2/34

(54) **Acetabular cup for use in a total hip prosthesis**
Hüftgelenkspfanne für eine Hüfttotalprothese
Cupule acétabulaire pour une prothèse totale de la hanche

(30) Priority: 08.08.1990 GB 9017403
(43) Date of publication of application: 26.02.1992
(73) Proprietor: HOWMEDICA INTERNATIONAL INC., Shannon Co. Clare (IE)
(72) Inventor: Ling, Robin Sidney Mackwood, Dittisham, Dartmouth TQ6 OHB (GB); Lawes, Peter, Maidenhead, Berkshire SL6 4DB (GB)
(74) Representative: Bridge-Butler, Alan James

(56) References cited:
- EP-A- 0 120 595
- EP-A- 0 225 819
- DE-A- 3 630 276
- US-A- 4 642 124

## Description

Loosening of acetabular cups which are held in place by a cement mantle is a major problem in total hip surgery and the object of the present invention is to produce an acetabular cup of this type which is less likely to loosen.

EP 0 120 595 discloses an acetabular cup to be held in place by a cement mantle. The cup includes a part-spherical element having an outer surface portion which decreases in diameter from its distal end to its proximal end and has spacers mounted at said proximal end to form a space. However the spacers known from EP 0 120 595 prevent the cup moving further into the cement mantle.

According to the present invention an acetabular cup for use in a total hip prosthesis includes a tapered element having an outer portion for location in a patient's pelvis and to be held in place in a cement mantle said outer surface portion tapering from its distal end to its proximal end and space creating means at said proximal end to form a space into which said tapered element can move subsequent to implantation to allow said element to move further in said cement mantle to engage itself to accommodate creep or movement of said cement.

Thus, should there be a tendency for the cup to become loose then a construction according to the invention is intended to relock the cup within the cement mantle, thus, if the bone remodels then the cement mantle will creep to re-adapt itself to the bone and the implant will re-engage in the cement mantle accommodating to the movement of the cement. If the bone cement is too highly stressed in places it will creep in order to relieve this stress and the implant will resettle.

Preferably the tapered element has a smooth surface in longitudinal directions so that when the implant resettles it will tend to lock due to the taper.

The tapered element is preferably substantially conical and can be provided with means to prevent rotation when located in the cement mantle.

Thus, the anti-rotation means can be provided on the element by multi-facets, flats, keyways or ridges or the element can have an oval or asymmetric cross-section.

It is important that the various anti-rotation means only extend in a direction or directions to prevent rotation and not in any spiral or circumferential direction that would tend to resist subsidence and relocking of the implant within the cement mantle.

Any movement when it must occur must not be between the cement and the bone and it is important therefore to protect the cement bone interface from disturbance.

In a preferred construction said space creating means are provided by enclosure means which have control means for allowing the proximal end to move further into a space in said enclosure means.

The space creating means can be in the form of a cup, one end of which is closed and in which the control means are in the form of a collar at a mid point or at the other end of the cup.

In another embodiment the walls of the cup enclosure means extend outwardly over the whole outer surface area of the tapered element.

These enclosure means can be made from material similar to bone cement material, for example an acrylic material such as polymethylmethacrylate to make it fully compatible with the bone cement and in effect provides a preformed cement mantle.

The outer surface of the element is made as smooth as possible, for example, by polishing a metal implant in order to reduce the friction and purchase between the implant and the cement. There must be no ridges, grooves, undercuts, matt finishes, or rough surfaces that would give a grip on the cement or the enclosure means. Ideally the only load transfer from the element to the cement or the enclosure means is through a comprehensive force normal to the interface surface between them.

The cup can have a bearing insert if desired, thus for example, the element can be in the form of a metal backing with a bearing insert within it. Alternatively the insert and fixation surface could be one and the same material. For example, a coating could be put on the bearing surface to articulate with a femoral stem or a coating could be put on the implant/cement interface portion merely to prevent adhesion and minimise friction with the cement.

The invention also includes a Total hip prosthesis incorporating an acetabular cup as set forth above.

The invention can be performed in various ways and some embodiments will now be described by way of example and with reference to the accompanying drawings in which :
Figure 1 is a diagrammatic view of an acetabular cup according to the invention;
Figure 2 is an end view of the cup shown in Figure 1;
Figure 3 is a diagrammatic view showing how the cup according to the invention can be implanted in the pelvis of a patient;
Figure 4 shows a cup carrying various anti-rotation features; and,
Figure 5 is a plan view of an acetabular cup which is slightly oval in cross-section to prevent rotation.

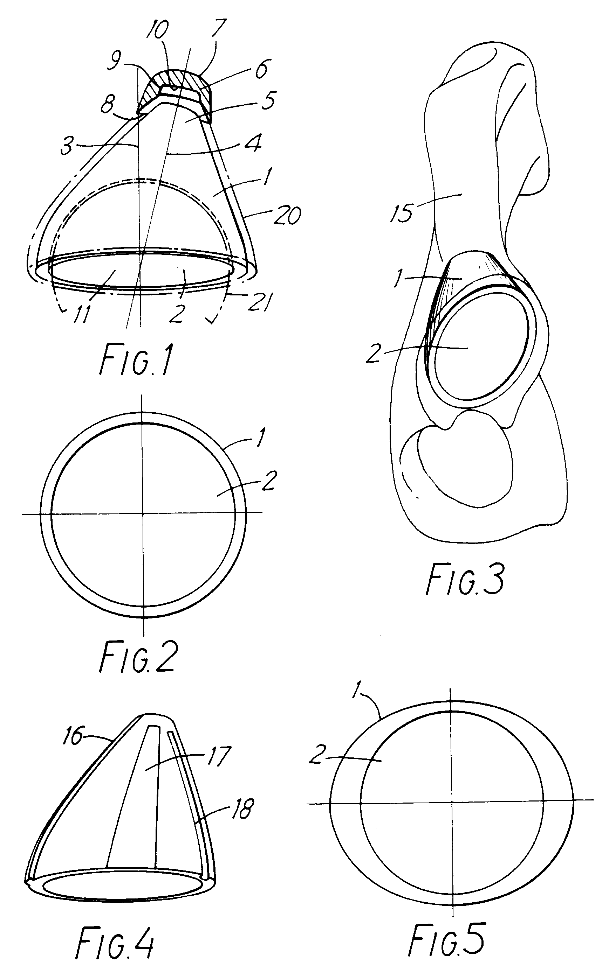

As shown in Figure 1 an acetabular cup for use in a Total hip prosthesis has an outer surface portion indicated by reference numeral 1. This portion consists of a tapered element which tapers from its distal end to its proximal end 5, having a smooth surface in longitudinal directions and being substantially conical. It will be seen that the outer surface of this element is not co-axial with a bearing surface 2 provided within the cup. The axis of the bearing surface is indicated by reference numeral 3 and the general axis of the conical element by reference numeral 4. The proximal end 5 of the element is housed within enclosure means in the form of an acrylic space creator 6. This device is provided as a cup having a closed end 7, an open end 8 and control means formed by a collar 9, the inner end of which is provided with a taper. This device is located by pushing it over the proximal end 5 of the conical element 1 so that there is a gap between the proximal end 5 and the inner surface 10 of the closed end of the cup thus creating a space between them. The space creator is added to the top of the acetabular cup as it is not desirable to bring the proximal end of the conical element to a sharp point and yet neither is it desirable that a blunt point can engage cement and resist the taper lock re-engagement to be described hereunder.

In an alternative construction the space creator 6 is formed to extend to envelop the whole of the outer surface 1 of the cup as shown in broken lines 20 in Figure 1. In this arrangement the control means can be provided by any convenient part of the engaging surface. The total enclosure of the outer surface of the cup allows the cup to move inwardly under load. The space creator is therefore in the form of a preformed sheath and as it is made from a material similar to bone cement material, for example polymethylmethacrylate, it is in effect another layer of cement but it is not secured to the cup. The ball of a femoral insert with the cup can be used to make up a total hip prosthesis is indicated in chain lines 21.

The thickness of the sheath can be as small as possible provided it is strong enough not to crack during implantation and, for example, it can be between 0.5 mm and 5 mm and is preferably about 1 mm for practical use.

It will be appreciated that the sheath is moulded separately and is shaped to fit a particular size of cup.

By preforming such a sheath there is a reduced adhesion between what is in effect the cement, provided by the sheath itself, and the outer surface of the implant thus not only providing better sliding of the implant within the cement mantle but relieving any loads which are likely to occur between the cement mantle and the bone.

It will be appreciated that the bearing surface 2 can house an inner bearing element or can be coated or made in any manner desired. In the arrangement shown, and as referred to above the angle of the outer end surface 11 of the acetabular cup is angled in relation to the axis 3 of the bearing surface 2.

Figure 3 shows how such an acetabular cup can be implanted in a patient's pelvis so that the conical element points along what is known as the sacral bar. On the pelvis there is a substantial bony bar passing from the acetabulum along the posterior medial portion of the ilium up to the joint with the sacrum. In order to prepare the acetabulum to accept the acetabular cup according to the invention part of the bone in the direction of this bony bar is reamed out to prepare a suitable tapering cavity. The acrylic void creator 6 and the mating tip of the conical element are placed co-axially inside this pre-prepared cavity pointing along the centre of the bony bar. In Figure 3 the bony bar is indicated by reference numeral 15. The acetabular cup is held in the pre-prepared cavity by cement which adheres to the bone and to the outer surface of the cup. Due to the smooth surface of the cup however, the adhesion between it and the cement is not strong and thus if there is any creep in the cement, caused for example by movement of the bone, the cup will tend to sink further into the cement and re-establish itself due to the taper. The acrylic space creator 6 (not shown in Figure 3) enables the cup to sink deeper into the cement with its proximal end being accommodated in the void 10 thus preventing splitting of the cement or resistance to inward movement.

It will be appreciated that there must be no ridges, grooves, undercuts, matt finishes, or rough surfaces that will give a grip on the cement and the outer surface of the implant is therefore deliberately finished to minimise friction and perches between it and the cement. If the implant is metal then the outer surface can be polished

With other known kinds of implant, when loosening starts in the cement it gradually gets worse and the attractive feature of the present construction is that movement tends to be self limiting, that is it is non-progressive. As the outside of the cup is not co-axial with the bearing surface there may be a tendency to turn and anti-rotation means can be provided as shown in Figures 4 and 5. Thus, in Figure 4 an acetabular cup is shown which is provided with three different types of, anti-rotation feature. Reference numeral 16 indicates a ridge, one or more of which can be provided on the outer surface, reference numeral 17 indicates a flat or facet, again one or more of which can be provided, and reference numeral 18 indicates a groove, again one or more of which can be incorporated.

It will be seen that the ridge, facet and groove all extend lengthways along the cup so that there is no tendency to resist subsidence and relocking of the implant within the cement mantle. It will be appreciated that all the movement when it must occur has to be between the implant and the cement and it is important to protect the cement bone interface from disturbance.

Figure 5 is a plan view of the cup in which the cross-section of the conical element is slightly oval.

The cup can be made from any suitable material, for example a synthetic plastics material, or a metal and, as mentioned above, can be made in any convenient form, for example with inserts or other features.

## Claims

1. An acetabular cup for use in a total hip prosthesis and which includes a tapered element having an outer surface portion (1) for location in a patient's pelvis and to be held in place by a cement mantle, said outer surface portion tapering from its distal end to its proximal end (5) and space creating means (6)(20) located at said proximal end (5) to form a space into which said tapered element (5) can move subsequent to implantation to allow said element to move further in said cement mantle to engage itself to accommodate creep or movement of said cement.

2. An acetabular cup as claimed in claim 1 in which said tapered element (1) has a smooth surface in longitudinal directions.

3. An acetabular cup as claimed in claim 2 in which said tapered element (1) is substantially conical.

4. An acetabular cup as claimed in claim 3 in which said substantially conical element (1) is provided with means (16)(17)(18) to prevent rotation when located in the fixation cement.

5. An acetabular cup as claimed in claim 4 in which said anti-rotation means are provided on the element by multifacets (17), flats (17), grooves (18), or ridges (16) or by said element having an oval or asymmetric cross-section.

6. An acetabular cup as claimed in any one of the preceding claims in which said space creating means are provided by enclosure means which have control means for allowing said proximal end (5) to move further into a space in said enclosure means (6)(20).

7. An acetabular cup as claimed in claim 6 in which said enclosure means are in the form of a cup (6), one end of which is closed and in which the control means are in the form of a collar (9) at a mid point or at the other end of the cup.

8. An acetabular cup as claimed in claim 6 in which the enclosure means are in the form of a cup (20), the walls of which extend outwardly over the whole outer surface area of the outer portion (1).

9. An acetabular cup as claimed in claim 6, claim 7 or claim 8 in which the enclosure means (6)(20) are made from a material similar to bone cement material.

10. An acetabular cup as claimed in any one of the preceding claims 1 to 9 in which it is provided with a bearing insert.

11. A total hip prosthesis incorporating an acetabular cup as set forth in any one of the preceding claims.

## Patentansprüche

1. Hüftgelenkspfanne zur Verwendung bei einer Hüftgelenks-Totalprothese und die ein verjüngtes Element mit einem äußeren Oberflächenteil (1) zur Positionierung in einer Hüfte eines Patienten einschließt, und um es durch einen Zementmantel in seiner Lage zu halten, wobei sich der besagte äußere Oberflächenteil von seinem distalen Ende zu seinem proximalen Ende (5) verjüngt, und einen Freiraum schaffende Einrichtungen (6) (20) an dem besagten proximalen Ende (5) angeordnet sind, um einen Freiraum zu bilden, in den sich das besagte verjüngte Element (5) nach einer Implantation bewegen kann, um es dem besagten Element zu gestatten, sich weiter in dem besagten Zementmantel zu bewegen, um sich zur Anpassung an ein Kriechen oder eine Bewegung des besagten Zements selbst in Eingriff zu bringen.

2. Hüftgelenkspfanne nach Anspruch 1, bei der das besagte verjüngte Element (1) in Längsrichtungen eine glatte Oberfläche aufweist.

3. Hüftgelenkspfanne nach Anspruch 2, bei der das besagte verjüngte Element (1) im wesentlich konisch ist.

4. Hüftgelenkspfanne nach Anspruch 3, bei der das besagte, im wesentlichen konische Element (1) mit Einrichtungen (16) (17) (18) versehen ist, um eine Drehung zu verhindern, wenn es im Fixationszement positioniert ist.

5. Hüftgelenkspfanne nach Anspruch 4, bei der die besagte Anti-Dreheinrichtungen auf dem Element durch Vielfachfacetten (17), Abflachungen (17), Nuten (18) oder Grate (16), oder dadurch vorgesehen sind, daß das besagte Element einen ovalen oder asymmetrischen Querschnitt aufweist.

6. Hüftgelenkspfanne nach einem beliebigen der vorangehenden Ansprüche, bei der die besagten, einen Freiraum schaffenden Einrichtungen von Umhüllungseinrichtungen bereitgestellt werden, die Steuereinrichtungen aufweisen, um es dem besagten proximalen Ende (5) zu gestatten, sich weiter in einen Freiraum in den besagten Umhüllungseinrichtungen (6) (20) zu bewegen.

7. Hüftgelenkspfanne nach Anspruch 6, bei der die besagten Umhüllungseinrichtungen in Form eines Napfes (6) vorliegen, dessen eines Ende geschlossen ist, und bei dem die Steuereinrichtungen in Form eines Kragens (9) an einer mittleren Stelle oder am anderen Ende des Napfes vorliegen.

8. Hüftgelenkspfanne nach Anspruch 6, bei der die Umhüllungseinrichtungen in Form eines Napfes (20) vorliegen, dessen Wände sich nach außen über den gesamten äußeren Oberflächenbereich des äußeren Teils (1) erstrecken.

9. Hüftgelenkspfanne nach Anspruch 6, Anspruch 7 oder Anspruch 8, bei der die Umhüllungseinrichtungen (6) (20) aus einem dem Knochenzementmaterial ähnlichen Material bestehen.

10. Hüftgelenkspfanne nach einem beliebigen der vorangehenden Ansprüche 1 bis 9, bei der sie mit einem Trageinsatz versehen ist.

11. Hüftgelenks-Totalprothese enthaltend eine Hüftgelenkspfanne nach einem beliebigen der vorangehenden Ansprüche.

## Revendications

1. Cupule acétabulaire pour une prothèse totale de la hanche et comprenant un élément aminci possédant une partie superficielle extérieure (1) en vue d'une mise en place dans le bassin d'un malade et pour être maintenue en position par une enveloppe extérieure de ciment,ladite partie superficielle extérieure s'amincissant depuis son extrémité distale vers son extrémité proximale (5) et des moyens créant un espace (6) (20) situés à ladite extrémité proximale (5) pour former un espace dans lequel ledit élément aminci (5) peut se déplacer ultérieurement à une implantation pour permettre audit élément de se déplacer encore dans ladite enveloppe extérieure de ciment pour venir au contact de celle-ci afin de tenir compte d'un glissement ou d'un déplacement dudit ciment.

2. Cupule acétabulaire selon la revendication 1, dans laquelle ledit élément aminci (1) présente une surface lisse dans des directions longitudinales.

3. Cupule acétabulaire selon la revendication 2, dans laquelle ledit élément aminci (1) est sensiblement conique.

4. Cupule acétabulaire selon la revendication 3, dans laquelle ledit élément sensiblement conique (1) est muni de moyens (16) (17) (18) pour empêcher une rotation lorsqu'il est disposé dans le ciment de fixation.

5. Cupule acétabulaire selon la revendication 4, dans laquelle lesdits moyens s'opposant à une rotation sont prévus sur l'élément par des facettes multiples (17), des méplats (17), des gorges (18), ou des nervures (16) ou par ledit élément présentant une section transversale ovale ou asymétrique.

6. Cupule acétabulaire selon l'une quelconque des revendications précédentes, dans laquelle lesdits moyens créant un espace sont constitués par des moyens formant une enceinte qui possèdent des moyens de commande pour permettre à ladite extrémité proximale (5) de se déplacer davantage dans un espace dans lesdits moyens formant enceinte (6) (20).

7. Cupule acétabulaire selon la revendication 6, dans laquelle lesdits moyens formant enceinte sont sous la forme d'une cuvette (6), dont une extrémité est fermée et dans laquelle les moyens de commande sont sous la forme d'un collier (9) en un point médian ou à l'autre extrémité de la cupule.

8. Cupule acétabulaire selon la revendication 6, dans laquelle les moyens formant enceinte sont sous la forme d'une cuvette (20), dont les parois s'étendent extérieurement au-dessus de la totalité de la zone superficielle de la partie extérieure (1).

9. Cupule acétabulaire selon la revendication 6, la revendication 7 ou la revendication 8, dans laquelle les moyens formant enceinte (6) (20) sont réalisés à partir d'un matériau similaire à un matériau ciment osseux.

10. Cupule acétabulaire selon l'une quelconque des revendications précédentes 1 à 9, dans laquelle est prévu un insert d'appui.

11. Prothèse totale de la hanche comportant une cupule acétabulaire selon l'une quelconque des revendications précédentes.
